(19) Europäisches Patentamt
European Patent Office
Office européen des brevets

(11) **EP 3 451 990 B1**

(12) **EUROPEAN PATENT SPECIFICATION**

(45) Date of publication and mention
of the grant of the patent:
**10.01.2024 Bulletin 2024/02**

(21) Application number: **17723610.6**

(22) Date of filing: **04.05.2017**

(51) International Patent Classification (IPC):
*A61F 13/49* (2006.01)   *D04H 1/559* (2012.01)
*D04H 3/04* (2012.01)   *D04H 1/4382* (2012.01)
*B32B 3/10* (2006.01)   *B32B 5/02* (2006.01)
*B32B 5/12* (2006.01)   *B32B 5/26* (2006.01)
*B32B 7/08* (2019.01)   *B32B 7/12* (2006.01)
*B32B 7/14* (2006.01)   *B32B 25/10* (2006.01)
*B32B 27/08* (2006.01)   *B32B 27/10* (2006.01)
*B32B 27/12* (2006.01)   *B32B 27/32* (2006.01)
*B32B 29/00* (2006.01)   *B32B 29/02* (2006.01)

(52) Cooperative Patent Classification (CPC):
**D04H 1/43832; A61F 13/49011; A61F 13/4902;
B32B 3/10; B32B 3/28; B32B 3/30; B32B 5/022;
B32B 5/024; B32B 5/12; B32B 5/24; B32B 5/26;
B32B 7/08; B32B 7/12; B32B 7/14; B32B 15/043;**

(Cont.)

(86) International application number:
**PCT/US2017/030945**

(87) International publication number:
**WO 2017/192790 (09.11.2017 Gazette 2017/45)**

(54) **NONWOVEN WEB MATERIAL HAVING BONDING FAVORABLE FOR MAKING DIRECTIONAL STRETCH LAMINATE, AND DIRECTIONAL STRETCH LAMINATE**

VLIESMATERIAL MIT ZUR HERSTELLUNG VON DIREKTIONAL DEHNBAREM LAMINAT VORTEILHAFTER BINDUNG SOWIE DIREKTIONAL DEHNBARES LAMINAT

MATÉRIAU DE VOILE NON-TISSÉ PRÉSENTANT UNE LIAISON FAVORABLE À LA FABRICATION D'UN STRATIFIÉ À ÉTIRAGE DIRECTIONNEL, ET STRATIFIÉ À ÉTIRAGE DIRECTIONNEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **04.05.2016 US 201662331650 P**

(43) Date of publication of application:
**13.03.2019 Bulletin 2019/11**

(73) Proprietor: **The Procter & Gamble Company
Cincinnati, OH 45202 (US)**

(72) Inventors:
• **DESAI, Fred Naval
Cincinnati, Ohio 45202 (US)**
• **ISELE, Olaf Erik Alexander
Cincinnati, Ohio 45202 (US)**
• **MINOGUCHI, Ryo
Cincinnati, Ohio 45202 (US)**
• **TRYGIER, Jill
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
**US-A1- 2002 016 122      US-A1- 2004 192 140
US-A1- 2005 136 771      US-A1- 2006 246 803
US-A1- 2013 306 226      US-A1- 2015 088 088**

**(Cont. next page)**

(52) Cooperative Patent Classification (CPC): (Cont.)
    **B32B 15/08; B32B 15/10; B32B 15/12;**
    **B32B 15/14; B32B 21/042; B32B 21/06;**
    **B32B 21/08; B32B 21/10; B32B 25/10;**
    **B32B 27/08; B32B 27/10; B32B 27/12;**
    **B32B 27/32; B32B 29/005; B32B 29/02;**
    **D04H 1/559; D04H 3/04;** B32B 2255/10;
    B32B 2255/205; B32B 2262/02; B32B 2262/0253;
    B32B 2262/0261; B32B 2262/0276; B32B 2270/00;
    B32B 2307/306; B32B 2307/51; B32B 2307/54;
    B32B 2307/546; B32B 2307/714; B32B 2307/734;
    B32B 2439/70; B32B 2555/02; B32B 2597/00;

D04H 1/43828

**Description**

BACKGROUND OF THE INVENTION

In recent years populations in many developed countries have shifted toward middle-aged and older demographic groups. These demographic groups represent markets with relatively increased demands for products and services addressed to concerns associated with aging.

One such concern is adult urinary incontinence. Urinary incontinence can result from or be exacerbated by a variety of health conditions, or even normal experiences such as childbearing.

Disposable absorbent pants for persons suffering from urinary incontinence have been marketed for a number of years. These products have traditionally been very similar to disposable baby diapers or disposable children's training pants, the main difference being size. One design type is known as the "belted" or "balloon" type pant, which is formed of a broad belt that encircles the wearer's waist and lower torso, bridged by a structure that connects front and rear belt portions through the wearer's crotch area. The crotch structure includes an absorbent structure designed to receive, contain and retain urine until the time the pant is changed. The belt is typically formed of a stretch laminate material.

Due to their design and method of manufacture, the products may visually resemble a disposable baby diaper or training pant, rather than an ordinary undergarment. The crotch structure may tend to be bulky as a result of the presence of absorbent materials. The structure may have the appearance of a mass-produced disposable article, like a disposable child diaper. The belt structure, typically formed of a stretch laminate material, may also have a bulky, mass-produced, diaper-like appearance.

This unfortunate resemblance has been a source of anxiety and discomfort for users. The bulk may cause outer clothing to fit poorly, or make it visibly obvious that an absorbent undergarment is being worn. Many users may be unhappy using products that may be associated with aging and loss of control of bodily functions.

In these circumstances, any improvement to traditional designs and materials for adult incontinence pants, that is efficient for manufacturing while providing an appearance and feel more closely resembling those of an ordinary undergarment, may provide competitive advantages to the manufacturer thereof.

Stretch laminates comprising one or more elastic members sandwiched between two web layers are disclosed in US2005/0136771 A1, US2002/0016122 A1, US2015/0088088 A1 and US2013/0306226 A1.

BRIEF DESCRIPTION OF THE DRAWINGS

Fig. 1 is a perspective view of one example of a balloon pant.
Fig. 2 is a perspective view of another example of a balloon pant.
Fig. 3 is a schematic plan view of a balloon pant precursor structure, prior to joining of the front and rear sections of the belt.
Fig. 4 is a schematic, exploded perspective view of components of a belt.
Fig. 5A is a plan view depiction of an example of a bond pattern.
Fig. 5B is a plan view depiction of another example of a bond pattern.
Fig. 5C is a plan view depiction of another example of a bond pattern.
Figs. 5D-5I are enlarged plan view depictions of additional examples of bond patterns.
Fig. 6 is an enlarged view of a portion of the bond pattern depicted in Fig. 5A.
Fig. 7 is a plan view of a portion of a stretch laminate bearing an example of a bond pattern, the laminate depicted in a stretched condition.
Fig. 8 is a plan view of the portion of stretch laminate depicted in Fig. 7, following elastic contraction of elastic members along a stretch direction, and formation of ruffles or gathers.
Fig. 9 is a cross section of a portion of the stretch laminate depicted in Fig. 8, taken along line 9-9 in Fig. 8.
Fig. 10 is a cross section of a portion of the stretch laminate depicted in Fig. 8, taken along line 10-10 in Fig. 8.

DETAILED DESCRIPTION OF EXAMPLES

Definitions

"Cross direction" (CD) - with respect to the making of a nonwoven web material, the nonwoven material itself, a laminate thereof, or an article in which the material is a component, refers to the direction along the material substantially perpendicular to the direction of forward travel of the material through the manufacturing line in which the material and/or article is manufactured.

Throughout the present description, a material or composite of materials is considered to be "elastic" or "elastomeric" if, when a biasing force is applied to the material, the material or composite can be extended to an elongated length of at least 150% of its original relaxed length (*i.e.* can extend at least 50%), without rupture or breakage which substantially damages the material or composite, and when the force is removed from the material or composite, the material or composite recovers at least 40% of such elongation. In various examples, when the force is removed from an elastically extensible material, the material or composite may recover at least 60% or even at least 80% of its elongation.

The "stretch direction" of a stretch laminate is the direction along which the laminate will most readily undergo elastic stretch and contraction. In a stretch laminate in which one or more elastic members are incorporated into the laminate while in a pre-strained condition, the stretch direction is the direction along which the elastic member(s) are pre-strained. The "trans-stretch direction" of a stretch laminate is the direction perpendicular to the stretch direction.

"Film" means a skin-like or membrane-like layer of material formed of one or more polymers, which does not have a form consisting predominately of a web-like structure of consolidated polymer fibers and/or other fibers.

"Lateral" - with respect to a pant and its wearer, refers to the direction generally perpendicular with the wearer's standing height, or the horizontal direction when the wearer is standing.

For purposes herein, the length (L) and width ($W$) of a bond shape are the trans-stretch direction and stretch direction (SD) dimensions, respectively, of a rectangle having the least possible area while entirely circumscribing the bond shape, and having two of its sides aligned parallel with the stretch direction SD.

"Longitudinal" - with respect to a pant and its wearer, refers to the direction generally parallel with the wearer's standing height, or the vertical direction when the wearer is standing. "Longitudinal" is also the direction generally parallel to a line extending from the midpoint of the front waist edge to the midpoint of the rear waist edge.

"Machine direction" (MD) - with respect to the making of a nonwoven web material, the nonwoven material itself, or a laminate thereof, refers to the direction along the material or laminate substantially parallel to the direction of forward travel of the material or laminate through the manufacturing line in which the material or laminate is manufactured.

"Machine direction bias," with respect to the fibers forming a nonwoven web, means that a majority of the fibers, as situated in the web and unstretched, have lengths with machine direction vector components that are greater than their cross direction vector components.

A "nonwoven" is a manufactured sheet or web of directionally or randomly oriented fibers which are first formed into a batt and then consolidated and bonded together by friction, cohesion, adhesion or one or more patterns of bonds and bond impressions created through localized compression and/or application of pressure, heat, ultrasonic or heating energy, or a combination thereof. The term does not include fabrics which are woven, knitted, or stitchbonded with yarns or filaments. The fibers may be of natural and/or man-made origin and may be staple and/or continuous filaments or be formed in situ. Commercially available fibers have diameters ranging from less than about 0.001 mm to more than about 0.2 mm and they come in several different forms: short fibers (known as staple, or chopped), continuous single fibers (filaments or monofilaments), untwisted bundles of continuous filaments (tow), and twisted bundles of continuous filaments (yarn). Nonwoven fabrics can be formed by many processes including but not limited to meltblowing, spunbonding, spunmelting, solvent spinning, electrospinning, carding, film fibrillation, melt-film fibrillation, airlaying, dry-laying, wetlaying with staple fibers, and combinations of these processes as known in the art. The basis weight of nonwoven fabrics is usually expressed in grams per square meter (gsm).

"z-direction," with respect to a web, means generally orthogonal or perpendicular to the plane approximated by the web along the machine and cross direction dimensions.

The invention is defined by the appended claims. Insofar as the term invention or example is used in the following, or features are presented as being optional, this should be interpreted in such a way that the only protection sought is that of the invention claimed. Reference(s) to "example(s)" throughout the description which are not under the scope of the appended claims merely represent possible exemplary executions and are not part of the present invention.

Although examples of the structure of the invention are described herein as used to form the belt of a belt- or balloon-type absorbent pant, it will be appreciated that examples may be used to form other components of pants, diapers, other wearable articles, and other products as well.

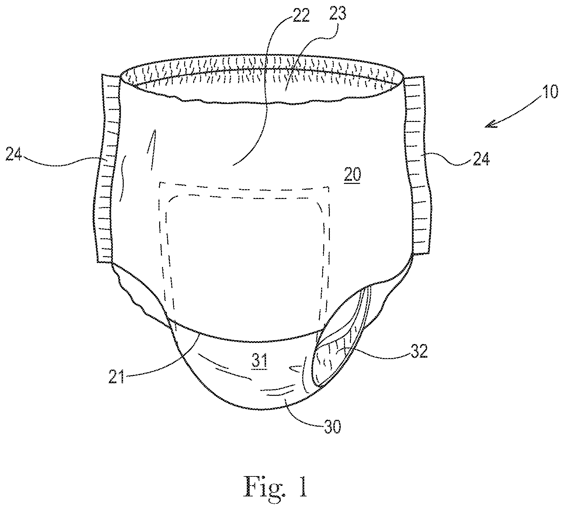

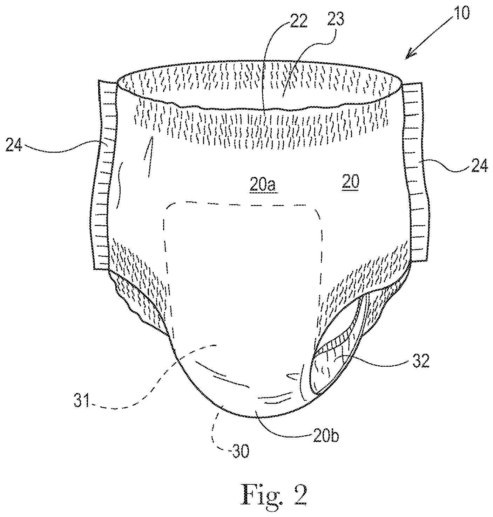

Figs. 1 and 2 depict examples of belt- or balloon-type absorbent pants 10. The structure may include a belt portion 20 and a central chassis 30. Central chassis 30 may include any combination of components found in disposable diapers and absorbent pants, including but not limited to a liquid impermeable backsheet 31, a liquid permeable topsheet (not shown), an absorbent core structure (not shown), and elasticized barrier cuffs 32. Examples and descriptions of components and configurations of a central chassis may be found in U.S. Pat. App. Ser. No. 13/764,945, wherein the chassis described includes components and features that may be included in central chassis 30.

In the example shown in Fig. 1, belt portion 20 stops short of the crotch region 12 of the pant, at lower edge 21; this configuration is sometimes called a "multipiece" configuration. In the example shown in Fig. 2, belt portion 20 is part of an outer structure that includes a belt portion 20a encircling the wearer's waist, an outer wrap portion 20b that overlies the central chassis to the outside thereof and wraps thereabout through the crotch region; this configuration is sometimes called a "unibody" configuration. The outer wrap portion 20b may be formed of a layer of nonwoven web, which also may serve as the outer layer of the belt portion 20a. The belt portion 20 may have front and rear portions 22, 23, which are joined together at seams 24.

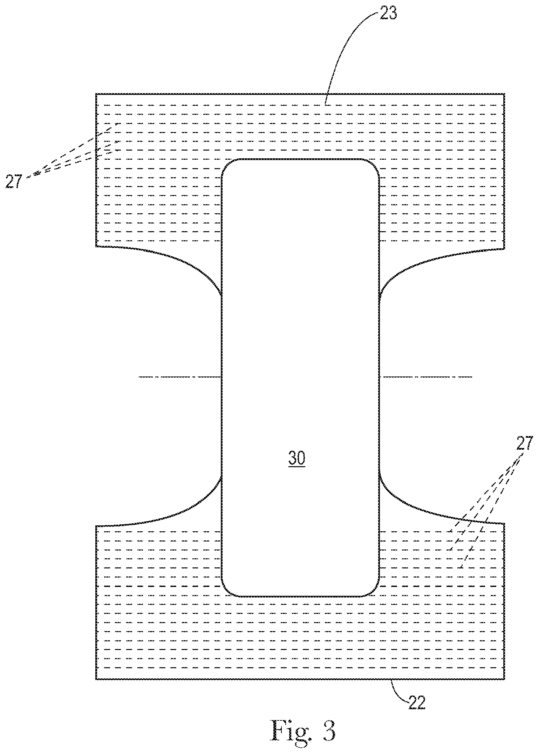

Fig. 3 schematically depicts a structure that is the precursor to a pant such as depicted in Fig. 2, prior to joining of front and rear portions 22, 23 at seams 24 as depicted in Figs. 1A and 1B. Central chassis 30 overlies front and rear portions 22, 23 to the inside thereof.

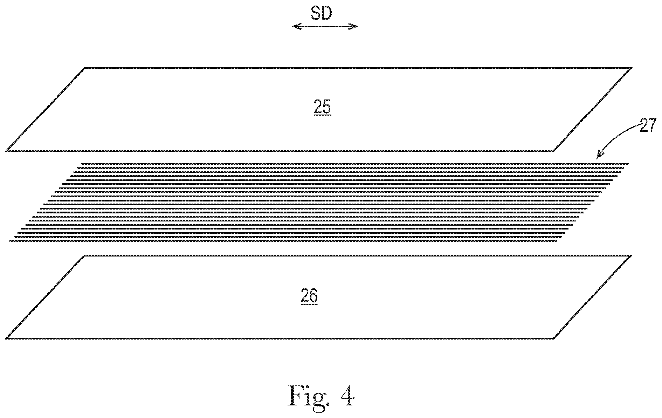

Referring to Fig. 4, a belt portion 20 may be formed of layers of nonwoven web 25, 26, which respectively form inner and outer layers of the belt. Suitable nonwoven web materials that may be useful in the present invention also include, but are not limited to, spunbond, spunlaid, meltblown, spunmelt, solvent-spun, electrospun, carded, film fibrillated, melt-film fibrillated, air-laid, dry-laid, wet-laid staple fibers, and other and other nonwoven web materials formed in part or in whole of polymer fibers, as known in the art. The nonwoven web may be formed predominately of polymeric fibers. In some examples, suitable non-woven fiber materials may include, but are not limited to polymeric materials such as polyolefins, polyesters, polyamide, or specifically, polypropylene (PP), polyethylene (PE), poly-lactic acid (PLA), polyethylene terephthalate (PET) and/or blends thereof. In some examples, the fibers may be formed of PP/PE blends such as described in U.S. Pat. No. 5,266,392, the disclosure of which is incorporated by reference herein. Nonwoven fibers may be formed of, or may include as additives or modifiers, components such as aliphatic polyesters, thermoplastic polysaccharides, or other biopolymers. Further useful nonwovens, fiber compositions, formations of fibers and nonwovens and related methods are described in U.S. Pats. Nos. 6,645,569; 6,863,933 and 7,112,621; and in co-pending U.S. patent application Ser. Nos. 10/338,603; 10/338,610; and 13/005, 237.

The individual fibers may be monocomponent or multicomponent. The multicomponent fibers may be bicomponent, such as in a core-and-sheath or side-by-side arrangement. Often, the individual components comprise polyolefins such as polypropylene or polyethylene, or their copolymers, polyesters, thermoplastic polysaccharides or other biopolymers.

According to one example, the nonwoven may comprise a material that provides good recovery when external pressure is applied and removed. Further, according to one example, the nonwoven may comprise a blend of different fibers selected, for example from the types of polymeric fibers described above. In some embodiments, at least a portion of the fibers may exhibit a spiral curl which has a helical shape. According to one example, the fibers may include bicomponent fibers, which are individual fibers each comprising different materials, usually a first and a second polymeric material. It is believed that the use of side-by-side bi-component fibers is beneficial for imparting a spiral curl to the fibers.

In order to enhance softness perceptions of the laminate, nonwovens may be treated by hydrojet impingement, which may also be known as hydroenhancement, hydroentanglement or hydroengorgement. Such nonwovens and processes are described in, for example, U.S. Pats. Nos. 6,632,385 and 6,803,103, and U.S. Pat. App. Pub. No. 2006/0057921.

Other examples of nonwoven web that may be useful in the present laminate may be an SMS web (spunbond-meltblown-spunbond web) made by Avgol Nonwovens LTD, Tel Aviv, Israel, under the designation XL-S70-26; an SSS (spunbond-spunbond-spunbond) web made by Pegas Nonwovens AS in Znojmo, Czech Republic, under the designation 18 XX 01 00 01 00 (where XX = the variable basis weight); an SSS web made by Gulsan Sentetik Dok San VE TIC AS, in Gaziantep, Turkey, under the designation SBXXFOYYY (where XX = the variable basis weight, and YYY = the variable cross direction width); an HESB (hydroenhanced spunbond) web made by First Quality Nonwovens Inc., in Hazelton, Pennsylvania, under the designation SEH2503XXX (where XXX = the variable cross direction width); and a bicomponent SS web.

A nonwoven web useful as a component to form one or both of layers 25, 26 may be bonded in a pattern of bonds. A batt of loose, e.g., spunlaid, fibers may be passed through the nip between a pair of calender bonding rollers and thereby consolidated and bonded in a pattern of bonds, to add tensile strength and dimensional stability, converting the batt of loose fibers to a coherent and useable nonwoven web material. The bonding include a pattern of thermal bonds. Thermal bonds may be formed by supplying one or both of the calender rollers or accompanying equipment with a source of heating energy that functions to heat the fibers and cause them to melt and fuse beneath bonding projections in the nip between the calender bonding rollers. One or both of the rollers may be machined, etched or otherwise formed to have a pattern of shaped bonding projections extending radially outward from the cylindrical surface of the roller. When the rollers are maintained in suitably close proximity with their axes in parallel, the batt of fibers passing therebetween will be subjected to pressure concentrated in the nip beneath the bonding projections, and fibers passing through the nip and beneath the bonding projections will be deformed and at least partially fused (by application of heating energy), to form bonds. Each bond will have a shape, and the bonds will have a pattern and spacing, substantially corresponding to the shape, pattern and spacing of the bonding projections on the calender bonding roller.

Referring to Fig. 4, layers of nonwoven web 25, 26 may sandwich one or more elastic members such as a plurality of strands 27 of an elastomeric material, such as an elastane (for example, LYCRA HYFIT fiber, a product of Invista, Wichita, Kansas). Layers of nonwoven web 25, 26 may be joined together about elastic strands 27 by adhesive deposited between the layers, by thermal bonds, by compression bonds, or by a combination thereof. In other examples, the one or more elastic members may be strips or a section of film formed of elastomeric material.

The elastic members can also be formed from various other materials, such as but not limited to, rubbers, styrene ethylbutylene styrene, styrene ethylene propylene styrene, styrene ethylene ethylene propylene styrene, styrene butadiene styrene, styrene isoprene styrene, polyolefin elastomers, elastomeric polyurethanes, and other elastomeric materials known in the art, and combinations thereof. In some embodiments, the elastic members can be extruded strand elastics with any number of strands (or filaments). Elastic strands, if used, may be selected to have a decitex ranging from 50 to 2000, or any integer value for any decitex value in this range, or any range formed by any of these integer values. The elastic members may be in a form of film. Examples of films have been described extensively in prior patent applications (*see*, for example, U.S. Pat. App. Pub. No. 2010/0040826). The film may be created with a variety of resins combined in at least one of several sublayers, the latter providing different benefits to the film. Elastic members may also be in the form of scrim, strips or sections of tape of elastomeric material with their longer dimensions oriented along the stretch direction.

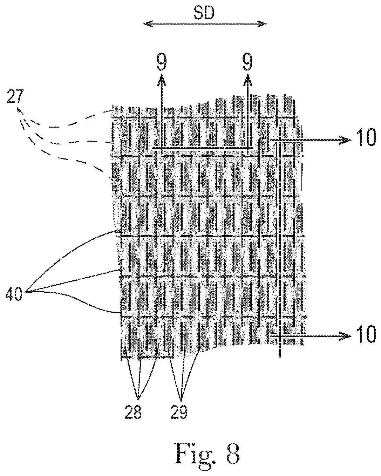

During manufacture of the belt structure, the one or more elastic members such as elastic strands 27, may be pre-strained lengthwise by a desired amount as they are being incorporated into the belt structure. Upon subsequent relaxation of the belt, the one or more elastic members" such as elastic strands 27, will contract toward their unstrained lengths. Referring to Figs. 8-10, this causes the layers of nonwoven material 25, 26 to gather and form ruffles or gathers having ridges 28 (shown in Fig. 8 as light areas) and valleys 29 (shown in Fig. 8 as dark areas) generally transverse to the lengths of the elastic strands 27. The direction of this strain corresponds with the stretch direction of the laminate.

The size(s) and shape(s) of the ruffles or gathers will be affected, and may be manipulated, by design of the pattern of joined portions and/or bonding between the layers of nonwoven web 25, 26, with respect to each other and with respect to elastic strands 27.

In one example, a stretch laminate may incorporate elastic strands 27 as the elastic stretch mechanism. Elastic strands 27 may have adhesive applied to them prior to lamination (e.g., by a strand coating process), such that, when the web layers 25, 26 are brought together to sandwich the strands, the applied adhesive causes the web layers to be adhered about the strands to form the stretch laminate. The adhesive applied to the elastic strands may be the only adhesive used to hold the laminate together. Alternatively, or in addition, adhesive may be deposited upon one or both layers 25, 26 prior to lamination, and may be deposited in a pattern. Examples of methods for applying patterned deposits of adhesive to a nonwoven web substrate to enable manufacture of an elasticized laminate are described in U.S. Pat. No. 8,186,296. In one example, the adhesive pattern selected may be effected by design of a correspondingly designed roller. The pattern of adhesive to be applied may be designed to affect the size(s) and shape(s) of the ruffles or gathers. The layers 25, 26 may be adhesively joined and/or bonded to each other at the locations of adhesive deposits, and remain unjoined or unbonded, or free, of each other at other locations, such that they may move and shift slightly relative each other as the laminate is moved and stretched, as during wear of the article.

When bonding of one or both of layers 25, 26 is effected using thermal calender bonding, the joining and/or bonding pattern may be designed to affect the size(s) and shapes of the ruffles or gathers.

Patterned thermal bonding tends to enhance machine-direction and cross-direction strength and dimensional stability of the resulting bonded nonwoven web, which has benefits in downstream converting and processing operations, and adds tensile strength and robustness to a product in which the web is to form a component.

However, thermal bonding also generally increases the stiffness of the resulting bonded nonwoven web. This may have adverse effects on the product consumer's perception of tactile softness of the product surfaces. For example, if the web is used as a layer of a belt structure of a pant product, stiffness imparted to the web may cause the consumer to negatively perceive the belt layer as stiff- or rough-feeling. For this reason, in some circumstances it may be desired to limit bond area to less than 8%. It has been discovered, however, that imparting certain features as described herein to the bond pattern of a web to be used in a stretch laminate can mitigate the negative effects of stiffening the web, while providing advantages in addition to tensile strength.

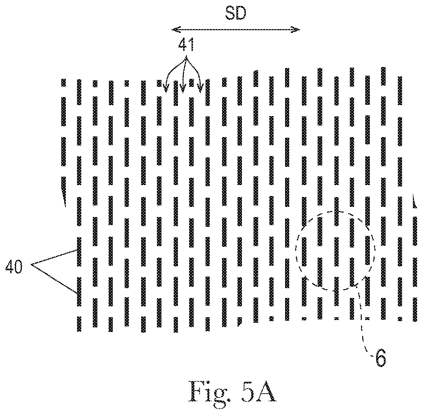

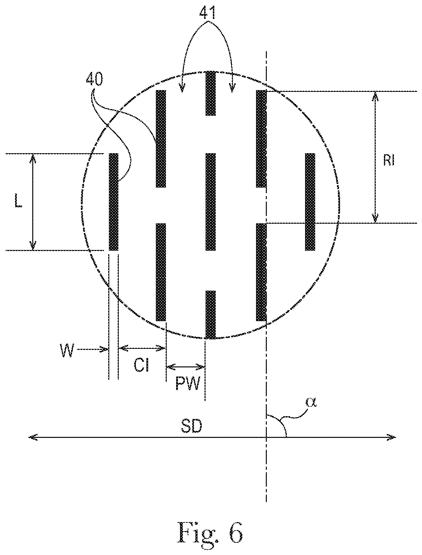

Referring to Figs. 5A and 6, a thermal bonding pattern may include a plurality of individual bonds 40 having elongate shapes having a length dimension Z measured along a trans stretch direction (perpendicular to the stretch direction *SD*) and a width dimension *W* measured along a direction parallel with the stretch direction *SD*. The bond shapes and arrangement on the nonwoven will substantially correspond with the shapes and arrangement of the radially outwardmost surfaces of the bonding projections (sometimes called a "bonding pins") on the calender roller used to create the bonds. The pattern may be either regular or irregular, along one or both of two perpendicular directions. Columns of individual bonds may be located at column repeat interval distances *CI*. Rows of individual bonds may be located at row repeat interval distances *RI*. Unbonded pathways 41 may be present between columns of bonds, and may extend indefinitely, or past only a plurality of rows, without interruption. Column angle $\alpha$ is the angle at the intersection between a line parallel with the stretch direction, and a line bordering an unbonded pathway 41 (tangent to the shapes in a column of bond shapes). Each unbonded pathway 41 has a width PW, measured along a direction parallel to the stretch direction. Width PW is the distance between two respective parallel lines bordering the unbonded pathway 41 (respectively tangent to the shapes in two adjacent columns of bond shapes), in which no portions of any bonds appear. Figs. 5A and 6 are not to be deemed limiting or exclusive examples of suitable bonding patterns contemplated herein.

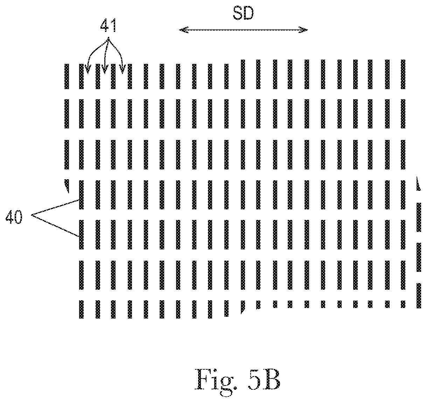

Additional, alternative but non-limiting, examples are depicted in Figs. 5B - 5I.

It has been found that orienting stiffening columns of bonds such that they run substantially perpendicular to the stretch direction *SD* (such that unbonded pathways 41 also run substantially perpendicular to and for a substantial length along the stretch direction), beneficial effects upon formation of ruffles or gathers in layers 25 and/or 26 may be achieved. Within the region occupied by a bond 40 in a nonwoven web layer 25 and/or 26, the constituent fibers of the web have been melted, deformed, highly consolidated and fused together. As a result, within the bond shape the structure is stiffened relative the surrounding unbonded areas. The stiffened area will tend to be more resistive to bending and flexing than the surrounding unbonded areas. Conversely, the unbonded areas (comprising undeformed and unbonded fibers) will tend to more easily bend and flex relative the stiffened areas within the bonds. Thus, the web will more easily bend and flex along an unbonded pathway 41, while columns of bonds 40 will tend to remain relatively resistant to bending and flexing. These effects may be exploited via design of the bond pattern, to favorably affect formation of ruffles or gathers in the stretch laminate.

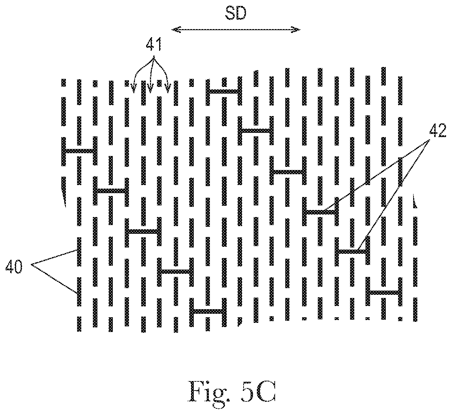

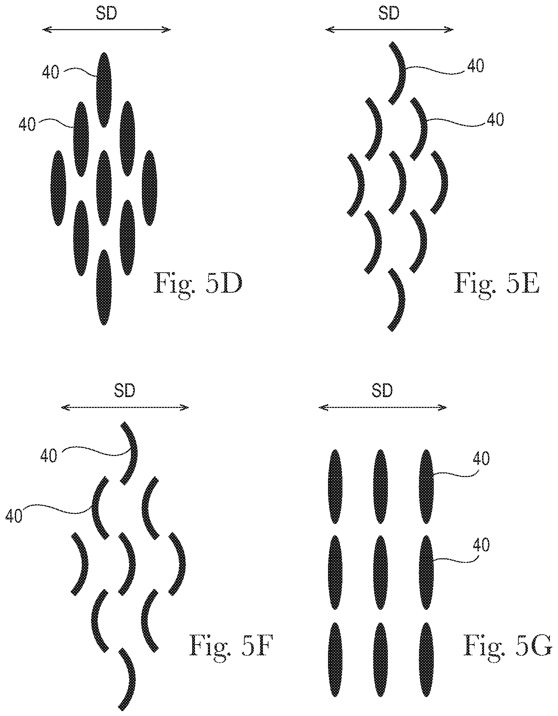

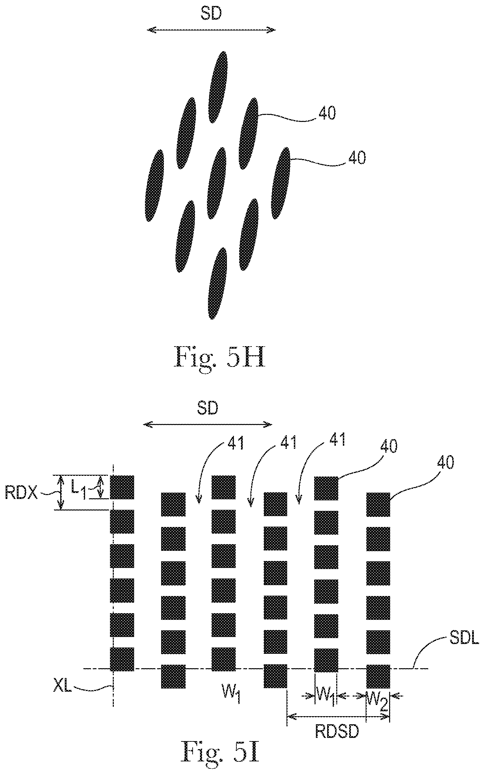

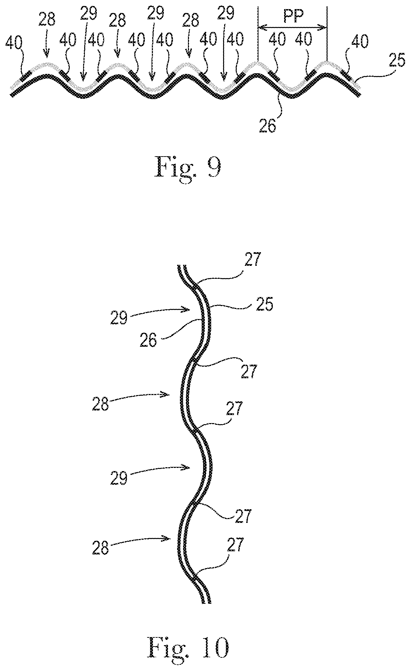

Referring to Figs. 5 and 9, bonds 40 will tend to cause the web layer 25 to resist bending in the bonded areas, such that peaks and floors of ridges 28 and valleys 29 are more likely to form along the more flexible unbonded areas such as unbonded pathways 41. As noted above, substantial bonding (*e.g.*, bonding area of greater than 4%) of nonwoven web layers of a stretch laminate may in some circumstances be undesirable because of perceived negative effects on stiffness and perceived softness of the material. However, when the bond pattern is configured as described herein, the stiffened (bonded) areas of the web may be effectively located away from the user's touch, by their locations on the sides of the ridges. The peaks of the ridges will be formed of unbonded, soft fibrous regions of the web, and protrude in the z-direction, imparting a soft tactile feel to the stretch laminate.

The shapes and dimensions of the bonds 40 is configured for beneficial impact not only on tactile softness, but on formation and size of the ruffles or gathers. Currently marketed belt- or balloon-style pants that include longitudinally spaced elastic strands as the belt stretch mechanism tend to have a bulky, puffy appearance that may not be deemed desirable for an adult product. It has been found that selecting spacing of the elastic strands in conjunction with spacing and dimensions of the bonds 40 in a bonded nonwoven layer 25 and/or 26 can work to greatly reduce the size of the ruffles or gathers, and also enhance regularity and consistency of size and shape. This helps impart a neat, low-bulk, cloth-like appearance to the stretch laminate.

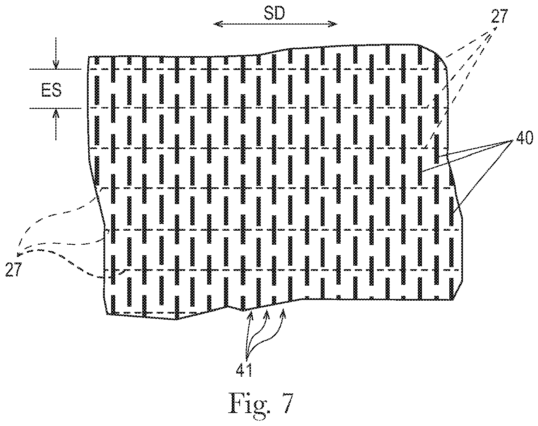

Referring to Figs. 7-10, straight, parallel and spaced elastic strands 27 may be incorporated into a stretch laminate in a pre-strained condition, along a stretch direction SD. Referring to Fig. 8, upon completion of manufacture of the stretch laminate, and elastic contraction of elastic strands 27 toward their unstrained lengths (as indicated by the large arrows), they will draw layers 25 and 26 and cause them to contract along the stretch direction and form ruffles or gathers having ridges 28 and valleys 29. The dimensions of the bond shapes and bond pattern may be coordinated with the trans-stretch spacing *S* of the elastic strands 27 for various advantages.

Alone or in combination with any other features described herein, bond shape length *L* may be selected so as to be no less than 33% of row repeat interval *RI*, more preferably no less than 40%, 50%, 60% and even more preferably no less than 70% of row repeat interval *RI*. This feature contributes to orderly and regular formation of gathers with peaks and valleys extending along a direction perpendicular to the stretch direction. It may be desired that bond shape length *L* be no greater than 90% of row repeat interval *RI*. This ensures that the bonds are discrete (not indefinitely long), and avoids imparting too much stiffness to the web along a direction perpendicular to the stretch direction *SD*.

Alternatively, or in combination with any other features described herein, bond shape width *W*, may be selected to be from 5% to 50% of column repeat interval *CI*, more preferably from 8% to 40%, and still more preferably from 10% to 30% of column repeat interval *CI*. This feature strikes a balance between providing an appropriate width for bond-stiffened regions that will resist bending and thereby form the sides of ridges 28, while leaving an appropriate width for non-bonded, non-stiffened regions that will bend or flex to form the peaks and floors of ridges 28 and valleys 29 of gathers in the web material.

Alternatively, or in combination with any other features described herein, the pattern of bonds may be arranged such that the total of unbonded pathway widths PW for the pattern over a stretch direction distance over which the pattern repeats is from 50% to 95% of the stretch direction repeat length, more preferably from 60% to 92% of the stretch direction repeat length, and still more preferably from 70% to 90% of the stretch direction repeat length.

Referring to Figs. 6 and 7, in combination with any other features described herein, bond row repeat interval *RI* may be related to trans-stretch elastic strand spacing *ES* such that *RI* is equal to or less than 2.0 times, more preferably 1.5 times, and even more preferably 1.0 times strand spacing *ES*. If *RI* is equal to or less than 1.0 x *ES*, lines of web flex extending along the stretch direction *SD* will be present in at least the same number and frequency as the number of elastic strands 27 present, promoting suitable flexibility along the trans-stretch direction. In conjunction with this feature, bond length *L* may be equal or less than 2.0 times, more preferably equal to or less than 1.5 times, and even more preferably equal to or less than 1.0 times strand spacing *ES*.

For purposes of reducing the overall size of the ruffles or gathers formed, and in conjunction with any combination of the features described herein, it may be desired that the trans-stretch spacing *ES* of the elastic strands 27 be no greater than 14 mm, more preferably no greater than 10 mm, even more preferably no greater than 7 mm, and still more preferably no greater than 5 mm. (Herein, trans-stretch spacing of adjacent elastic strands is to be understood to refer to the distance between their longitudinal axes, not the distance between their nearest outer surfaces.) Through experimentation it has been determined that limiting spacing of elastic strands 27 in this way has the effect of promoting formation of ruffles or gathers that are relatively small, thereby promoting or enhancing a cloth-like appearance in the stretch laminate. This effect may be complimented and amplified by incorporating other features of a bond pattern in one or both of the nonwoven web layers 25, 26 as described herein.

In combination with any of the other features described herein, column repeat interval $CI$ may be related to trans-stretch elastic strand spacing $ES$ such the $CI$ is no greater than 1.5 $ES$, more preferably no greater than 1.3 $ES$, 1.0 $ES$, 0.9 $ES$, and even more preferably no greater than 0.8 $ES$. This feature enables control of the size and regularity/uniformity of the ruffles or gathers formed relative the trans-stretch spacing of the elastic strands 27. If, for example, a stretch laminate has elastic strands spaced at $ES$ = 5.0 mm, this means that it may be desired that bond column repeat interval $CI$ be 7.5 mm or less, 6.5 mm or less, 5.0 mm or less, 4.5 mm or less, or even 4.0 mm or less. This dimension, along with the extent of pre-strain imparted to the elastic strands 27 during formation of the stretch laminate, will further impact the stretch-direction size of the ruffles or gathers. For stretch laminates as contemplated herein, pre-strain in the elastic strands in the range from 50% to 200% is envisioned. (Herein, the pre-strain percentage amount is expressed as the calculation of the length of a section of the strand in the pre-strained condition minus the length of the same section of the strand in its relaxed condition (stretch distance), divided by length of the same section of the strand in its relaxed condition, times 100%.) For example, assuming a level of pre-strain in the elastic strands sufficient to cause the laminate to contract upon relaxation to approximately half of its stretch-direction, fully-stretched length (*e.g.,* a pre-strain amount of approximately 100%), a bond column repeat interval $CI$ will promote formation of ruffles or gathers upon relaxation of the stretch laminate that have a peak-to-peak dimension $PP$ (*see* Fig. 9) of approximately half of bond column repeat interval $CI$. Under this circumstance, if bond column repeat interval $CI$ is 7.5 mm or less, formation of regular, uniform ruffles or gathers having a peak-to-peak dimension of 3.8 mm or less, may be promoted. This is the stretch-direction relaxed width of the ruffles or gathers. From construction of prototypes and observation it has been determined that uniform and regular ruffles or gathers of such width or smaller visually appear quite small and thereby substantially contribute to imparting a cloth-like appearance to the stretch laminate.

In combination with any of the other features described herein, a majority of the bonds 40 in the pattern may have shapes with an aspect ratio of length to width equal to or greater than 1.0, more preferably equal to or greater than 1.5, even more preferably equal to or greater than 2.0, still more preferably equal or greater than 2.5, and most preferably equal to or greater than 3.5. This feature will contribute to promoting formation of regular and uniform ruffles or gathers with ridges and valleys oriented in the trans-stretch direction.

Another characteristic of a bond pattern that can provide a way to effect control over formation of ruffles or gathers for purposes herein may be described with reference to Fig. 5I. A bond pattern will have a stretch direction repeat interval RDSD measured along a line SDL parallel with the stretch direction SD. A bond pattern will have a trans-stretch direction repeat interval RDX measured along a line XL perpendicular to the stretch direction SD. Bonds 40 included within the stretch direction repeat interval RDSD may have, *e.g.*, stretch direction widths W1, W2, W3, etc. Bonds 40 within the trans-stretch direction repeat interval RDX may have, e.g., trans-stretch direction lengths L1, L2, L3, etc. In a bond pattern suitable for purposes herein: a trans-stretch direction line XL may be identified, along which the ratio of the sum of the bond lengths (L1, L2, etc.) to the trans-stretch direction repeat interval (RDX) that includes the bond lengths, is greater than the ratio of the sum of the bond widths (W1, W2, etc.) to the stretch direction repeat interval (RDSD) that includes the bond widths, along any stretch direction line (SDL) along the pattern that can be identified. Expressed differently, in an example of a bond pattern suitable for purposes herein: a line XL perpendicular to the stretch direction can be identified, wherealong

$$(L1 + L2 + \text{etc.})/RDX > (W1 + W2 + \text{etc.})/RDSD,$$

for any line SDL that can be identified extending in the stretch direction. Expressed yet another way, a bond pattern in which a trans-stretch direction line along which bonds are arranged traverses a greater sum of bond lengths per unit line length along the web, than any sum of bond widths per unit line length crossed by any stretch direction line that can be identified. The effect of this configuration of bonds is substantially trans-stretch direction columnar stiffened regions alternating with substantially trans-stretch direction columnar flexible regions. This configuration tends to promote formation of ruffles or gathers with improved uniformity and regularity, with alternating ridges 28 and valleys 29 oriented along the flexible columnar regions, and sides or slopes along the stiffened columnar regions, as may be appreciated from Fig. 9. Assuming that the various examples of patterns depicted in Figs. 5A-5I are proportionally accurate as reproduced from the drawings originally prepared for submission herewith, it can be appreciated that each satisfies this condition (or will, with minor adjustment to proportions), although these are only non-limiting, non-exclusive examples.

The columns of bond shapes may be configured to be substantially perpendicular to the stretch direction *SD*, *e.g.*, in Fig. 6, column angle α is 90 degrees. It is not necessary that angle α is exactly 90 degrees, but it may be preferable that column angle α is from 70 to 110 degrees, more preferably from 80 to 100 degrees, even more preferably from 85 to 95 degrees, and most preferably from 87 to 93 degrees. This ensures orderly, regular formation of ruffles or gathers along a direction substantially perpendicular to the stretch direction.

A pattern that has unbonded pathways 41 that lie exactly along the cross-direction of the bonded nonwoven web material reflects a calender bonding roller with a corresponding arrangement of bonding projections. This arrangement pay increase the possibility for increased or undesired excess pressure beneath bonding projections that may result in non-uniform or defective bonds and/or roller and equipment wear, which may result from intermittent and rapid, step-wise changes in pressure in the nip resulting from intermittent absence and presence of bonding projections in the nip that may occur when trans-machine direction columns of bonding projections alternate with trans-machine direction unbonded pathways with no projections present on the cylindrical surface of the calender bonding roller.

Thus, when the machine direction of the bonded nonwoven web layer corresponds with the stretch direction, it may be desired that column angle α not be exactly 90 degrees. Alternatively or in addition, the calender bonding roller may include bonding projections that intermittently interrupt the unbonded pathways 41, resulting in formation of trans-stretch pathway interrupting bonds 42, as suggested in Fig. 5C. Interrupting bonds 42 reflect bonding projections that may be included to mitigate or eliminate the above-referenced rapid, step-wise changes in pressure in the nip between the calender bonding rollers.

The stretch direction SD of the stretch laminate may be parallel or perpendicular with the machine direction of manufacture of the nonwoven web layer components 25, 26. For nonwoven webs formed of continuously spun fibers, the fibers in the web often have a machine-direction bias as a result of the manner in which the fibers are spun and laid down on a moving belt. As a result of this machine-direction bias of the fibers, the nonwoven web may be imparted with anisotropic tensile strength properties, for example, machine-direction tensile strength is greater than cross-direction tensile strength. It has been determined, however, than when a bond pattern has a combinations of one or more features described above, the columns of bond shapes are substantially perpendicular to the machine direction, the rows of bond shapes are offset or staggered as suggested in Fig. 5A (in contrast to aligned as depicted in Fig. 5B), that anisotropy of tensile strength may be substantially reduced, and dimensional stability of the web may be substantially improved (particularly but without limitation, undesirable necking or neck-down behavior may be substantially reduced). As noted, tensile strength and dimensional stability of the nonwoven web are important for converting and processing operations, and for structural integrity and robustness of a product in which the nonwoven web is used as a component. Accordingly, it may be desired that the stretch direction of the stretch laminate be substantially parallel to (*i.e.*, substantially the same as) the machine direction of the nonwoven web layer bearing a bond pattern as described herein.

## Claims

1.    A stretch laminate having a stretch direction and a trans-stretch direction perpendicular to the stretch direction, comprising:

   a first web layer comprising a nonwoven web material bearing a pattern of thermal bonds comprising a plurality of bonds each having a length and a width, the length being oriented perpendicularly to the stretch direction and being greater than the width, for a majority of the bonds;
   a second web layer; and
   one or more elastic members disposed between the first web layer and the second web layer, wherein the first web layer, the second web layer and the one or more elastic members together form the stretch laminate;
   wherein the one or more elastic members have been joined with the first web layer and the second web layer while the one or more elastic members are pre- strained in the stretch direction;
   wherein, when the stretch laminate is in a relaxed condition, at least the first web layer comprises a plurality of gathers comprising elongate ridges and valleys oriented transversely to the stretch direction;
   wherein each ridge of the plurality of gathers has a peak and two sloped sides, and one or both of the sides of one or more of the ridges bears at least one of the plurality of bonds;
   wherein the one or more elastic members comprises a plurality of elastic strands disposed between the first web layer and the second web layer, with longitudinal axes being substantially parallel with the stretch direction and spaced apart in the trans-stretch direction by a strand spacing ES; and
   wherein the length of the bonds is no greater than the strand spacing ES.

2.    The stretch laminate of claim 1 wherein the strand spacing is no greater than 14 mm, more preferably no greater than 10 mm, even more preferably no greater than 7 mm, and still more preferably no greater than 5 mm.

3. The stretch laminate of either of claims 1 or 2, wherein the bonds in the plurality of bonds are arranged in regularly-spaced columns, the columns having a column repeat interval CI, and wherein the column repeat interval CI is no greater than 1.5 ES, more no greater than 1.3 ES, 1.0 ES, 0.9 ES, and even more preferably no greater than 0.8 ES.

4. The stretch laminate of any of the preceding claims wherein the bonds in the plurality of bonds have a substantially straight rod shape.

5. The stretch laminate of any of the preceding claims wherein the nonwoven web material has a machine direction and a cross direction perpendicular to the machine direction, and the stretch direction is substantially parallel with the machine direction.

6. The stretch laminate of any of the preceding claims wherein the nonwoven web material has a machine direction and comprises spunlaid fibers.

7. The stretch laminate of claim 6 wherein the spunlaid fibers have a machine direction bias.

8. The stretch laminate of claim 7 wherein the machine direction is substantially parallel with the stretch direction.

9. The stretch laminate material of any of claims 1-3 wherein the elastic strands have been pre-strained by an amount from 50% to 200% prior to their incorporation into the stretch laminate material.

10. A disposable absorbent pant having an elasticized belt about a waist region, the belt comprising the stretch laminate material of any of the preceding claims.


**Patentansprüche**

1. Dehnlaminat, das eine Dehnungsrichtung und eine Transdehnungsrichtung lotrecht zu der Dehnungsrichtung aufweist, umfassend:

eine erste Bahnschicht, umfassend ein Vliesbahnmaterial, das ein Muster von thermischen Bindungen trägt, umfassend eine Vielzahl von Bindungen, die jeweils eine Länge und eine Breite aufweisen, wobei die Länge lotrecht zu der Dehnungsrichtung ausgerichtet ist und größer als die Breite ist, für einen Großteil der Bindungen; eine zweite Bahnschicht; und
ein oder mehrere elastische Elemente, die zwischen der ersten Bahnschicht und der zweiten Bahnschicht angeordnet sind, wobei die erste Bahnschicht, die zweite Bahnschicht und das eine oder die mehreren elastischen Elemente zusammen das Dehnlaminat ausbilden;
wobei das eine oder die mehreren elastischen Elemente mit der ersten Bahnschicht und der zweiten Bahnschicht verbunden wurden, während das eine oder die mehreren elastischen Elemente in der Dehnungsrichtung vorgestreckt sind;
wobei, wenn sich das Dehnlaminat in einem entspannten Zustand befindet, mindestens die erste Bahnschicht eine Vielzahl von Raffungen umfasst, umfassend längliche Kämme und Vertiefungen, die querverlaufend zu der Dehnungsrichtung ausgerichtet sind;
wobei jeder Kamm der Vielzahl von Raffungen eine Spitze und zwei abgeschrägte Seiten aufweist und eine oder beide der Seiten eines oder mehrerer der Kämme mindestens eine der Vielzahl von Bindungen trägt;
wobei das eine oder die mehreren elastischen Elemente eine Vielzahl von elastischen Strängen umfassen, die zwischen der ersten Bahnschicht und der zweiten Bahnschicht angeordnet sind, wobei Längsachsen im Wesentlichen parallel zu der Dehnungsrichtung und in der Transdehnungsrichtung durch einen Strangabstand ES beabstandet sind; und
wobei die Länge der Bindungen nicht größer als der Strangabstand ES ist.

2. Dehnlaminat nach Anspruch 1, wobei der Strangabstand nicht mehr als 14 mm, mehr bevorzugt nicht mehr als 10 mm, noch mehr bevorzugt nicht mehr als 7 mm und ferner noch mehr bevorzugt nicht mehr als 5 mm, beträgt.

3. Dehnlaminat nach einem der Ansprüche 1 oder 2, wobei die Bindungen in der Vielzahl von Bindungen in regelmäßig beabstandeten Spalten angeordnet sind, wobei die Spalten ein Spaltenwiederholungsintervall CI aufweisen, und wobei das Spaltenwiederholungsintervall CI nicht mehr als 1,5 ES, mehr nicht mehr als 1,3 ES, 1,0 ES, 0,9 ES und noch mehr bevorzugt nicht mehr als 0,8 ES, beträgt.

**4.** Dehnlaminat nach einem der vorstehenden Ansprüche, wobei die Bindungen in der Vielzahl von Bindungen eine im Wesentlichen gerade Stabform aufweisen.

**5.** Dehnlaminat nach einem der vorstehenden Ansprüche, wobei das Vliesbahnmaterial eine Maschinenrichtung und eine Querrichtung lotrecht zu der Maschinenrichtung aufweist und die Dehnungsrichtung im Wesentlichen parallel zu der Maschinenrichtung ist.

**6.** Dehnlaminat nach einem der vorstehenden Ansprüche, wobei das Vliesbahnmaterial eine Maschinenrichtung aufweist und Spinnvliesfasern umfasst.

**7.** Dehnlaminat nach Anspruch 6, wobei die Spinnvliesfasern eine Maschinenrichtungsvorspannung aufweisen.

**8.** Dehnlaminat nach Anspruch 7, wobei die Maschinenrichtung im Wesentlichen parallel zu der Dehnungsrichtung ist.

**9.** Dehnlaminatmaterial nach einem der Ansprüche 1 bis 3, wobei die elastischen Stränge vor ihrer Beimischung in das Dehnlaminatmaterial um eine Menge von 50 % bis 200 % vorgestreckt wurden.

**10.** Einweg-Absorptionshöschen, das einen elastifizierten Gurt um einen Taillenbereich aufweist, der Gurt umfassend das Dehnlaminatmaterial nach einem der vorstehenden Ansprüche.

**Revendications**

**1.** Stratifié étirable présentant une direction d'étirement et une direction de trans-étirement perpendiculaire à la direction d'étirement, comprenant :

une première couche de bande comprenant un matériau de bande non tissée portant un motif de liaisons thermiques comprenant une pluralité de liaisons ayant chacune une longueur et une largeur, la longueur étant orientée perpendiculairement à la direction d'étirement et étant supérieure à la largeur, pour une majorité des liaisons ;
une seconde couche de bande ; et
un ou plusieurs éléments élastiques disposés entre la première couche de bande et la seconde couche de bande, dans lequel la première couche de bande, la seconde couche de bande et un ou plusieurs éléments élastiques forment ensemble le stratifié étirable ;
dans lequel un ou plusieurs éléments élastiques ont été joints à la première couche de bande et à la seconde couche de bande tandis qu'un ou plusieurs éléments élastiques sont précontraints dans la direction d'étirement ;
dans lequel, lorsque le stratifié étirable est dans une condition relaxé, au moins la première couche de bande comprend une pluralité de fronces comprenant des crêtes et vallées allongées orientées transversalement à la direction d'étirement ;
dans lequel chaque crête de la pluralité de fronces a un pic et deux côtés inclinés, et l'un ou les deux des côtés d'une ou plusieurs des crêtes porte au moins une de la pluralité de liaisons ;
dans lequel un ou plusieurs éléments élastiques comprennent une pluralité de fils élastiques disposés entre la première couche de bande et la seconde couche de bande, avec des axes longitudinaux qui sont sensiblement parallèles à la direction d'étirement et espacés dans la direction de trans-étirement par un espacement de fils ES ; et
dans lequel la longueur des liaisons n'est pas supérieure à l'espacement de fils ES.

**2.** Stratifié étirable selon la revendication 1, dans lequel l'espacement des fils n'est pas supérieur à 14 mm, plus préférablement n'excédant pas 10 mm, même plus préférablement n'excédant pas 7 mm, et encore plus préférablement n'excédant pas 5 mm.

**3.** Stratifié étirable selon l'une ou l'autre des revendications 1 ou 2, dans lequel les liaisons dans la pluralité de liaisons sont agencées en colonnes espacées régulièrement, les colonnes ayant un intervalle de répétition de colonne CI, et l'intervalle de répétition de colonne CI n'étant pas supérieur à 1,5 ES, plus précisément à 1,3 ES, 1,0 ES, 0,9 ES, et encore plus préférablement n'excédant pas 0,8 ES.

**4.** Stratifié étirable selon l'une quelconque des revendications précédentes dans lequel les liaisons dans la pluralité de liaisons ont une forme de tige sensiblement droite.

**5.** Stratifié étirable selon l'une quelconque des revendications précédentes dans lequel le matériau de bande non tissé a une direction de machine et une direction transversale perpendiculaire à la direction de machine, et la direction d'étirement est essentiellement parallèle à la direction de machine.

**6.** Stratifié étirable selon l'une quelconque des revendications précédentes dans lequel le matériau de bande non tissé a une direction de machine et comprend des fibres filées.

**7.** Stratifié étirable selon la revendication 6 dans lequel les fibres filées ont un biais dans la direction de machine.

**8.** Stratifié étirable selon la revendication 7, dans lequel la direction de machine est essentiellement parallèle à la direction d'étirement.

**9.** Matériau stratifié étirable selon l'une quelconque des revendications 1 à 3 dans lequel les fils élastiques ont été précontraints d'une quantité allant de 50 % à 200 % avant leur incorporation dans le matériau stratifié étirable.

**10.** Culotte absorbante jetable ayant une courroie élastique autour d'une région de taille, la courroie comprenant le matériau stratifié étirable selon l'une quelconque des revendications précédentes.

Fig. 1

Fig. 2

Fig. 3

Fig. 4

Fig. 5A

Fig. 5B

Fig. 5C

Fig. 5D

Fig. 5E

Fig. 5F

Fig. 5G

SD

Fig. 5H

SD

RDX

$L_1$

41   41   41   40   40

XL

$W_1$   $W_1$   $W_2$

SDL

RDSD

Fig. 5I

Fig. 6

Fig. 7

Fig. 8

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20050136771 A1
- US 20020016122 A1
- US 20150088088 A1
- US 20130306226 A1
- US 764945
- US 5266392 A
- US 6645569 B
- US 6863933 B
- US 7112621 B

- US 338603
- US 10338610 B
- US 13005 A
- US 237 A
- US 6632385 B
- US 6803103 B
- US 20060057921
- US 20100040826
- US 8186296 B